(19) Europäisches Patentamt
European Patent Office
Office européen des brevets

(11) Veröffentlichungsnummer : **0 098 591**
**B1**

(12) **EUROPÄISCHE PATENTSCHRIFT**

(45) Veröffentlichungstag der Patentschrift : **08.07.87**

(21) Anmeldenummer : **83106569.3**

(22) Anmeldetag : **05.07.83**

(51) Int. Cl.[4] : **C 07 D333/54**, C 07 D333/60, C 07 D409/06, C 07 D333/38, C 07 D307/68, C 07 D307/79, C 07 D213/80, A 01 N 43/06// C07D333/56, C07F9/38

(54) **Neue heterocyclische Verbindungen und diese enthaltende Rodentizide.**

(30) Priorität : **06.07.82 CH 4117/82**
**19.05.83 CH 2728/83**

(43) Veröffentlichungstag der Anmeldung : **18.01.84 Patentblatt 84/03**

(45) Bekanntmachung des Hinweises auf die Patenterteilung : **08.07.87 Patentblatt 87/28**

(84) Benannte Vertragsstaaten : **AT BE CH DE FR GB IT LI LU NL SE**

(56) Entgegenhaltungen :
**EP-A- 0 002 742**
**DE-A- 2 819 213**

(73) Patentinhaber : **F. HOFFMANN-LA ROCHE & CO. Aktiengesellschaft**
**CH-4002 Basel (CH)**

(72) Erfinder : **Klaus, Michael, Dr.**
**Am Hellenrain 6**
**D-7858 Weil/Rhein (DE)**
Erfinder : **Weiser, Harald, Dr.**
**Bürenweg 248**
**CH-4146 Hochwald (CH)**
Erfinder : **Loeliger, Peter, Dr.**
**Hauptstrasse 19**
**CH-4132 Muttenz (CH)**

(74) Vertreter : **Lederer, Franz, Dr. et al**
**Vanderwerth, Lederer & Riederer Patentanwälte Lucile-Grahn-Strasse 22**
**D-8000 München 80 (DE)**

**EP 0 098 591 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue heterocyclische Verbindungen der allgemeinen Formel

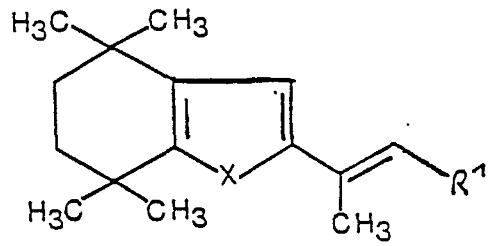

(I)

worin X —CH=CH—, —O— oder —S— ist ; $R^1$ ein Rest Ar—$R^2$ oder —CH=CH—C($CH_3$)=CH—$R^{21}$ ; Ar Phenyl, Pyridyl, Furyl oder Thienyl ; $R^2$ ein Rest —$CO_2R^3$, —C(O)$R^4$, —$CH_2OR^3$, $C_1$-$C_6$-Alkylsulfonyl oder Formyl ; $R^{21}$ ein Rest —$CO_2R^3$, —C(O)$R^4$, —$CH_2OR^3$ oder Formyl ; $R^3$ Wasserstoff oder $C_1$-$C_6$-Alkyl und $R^4$ Wasserstoff, Hydroxy, Amino, $C_1$-$C_6$-Alkylamino, di-($C_1$-$C_6$-Alkyl)amino oder $C_1$-$C_6$-Alkyl ist ; wobei mindestens ein Ring des Moleküls heterocyclisch ist.

Die Erfindung betrifft weiterhin ein Verfahren zur Herstellung der Verbindungen der Formel I sowie Rodentizide, die eine Verbindung der Formel I enthalten.

Der hier verwendete Ausdruck $C_1$-$C_6$-Alkyl bezieht sich auf geradkettige oder verzweigte Alkylgruppen mit 1-6, vorzugsweise 1-4 C-Atomen, wie Methyl, Aethyl, Propyl, Isopropyl, Butyl, sek.Butyl. Besonders bevorzugt sind Methyl und Aethyl.

Die Verbindungen der Formel I können erfindungsgemäss dadurch erhalten werden, dass man eine Verbindung der allgemeinen Formel

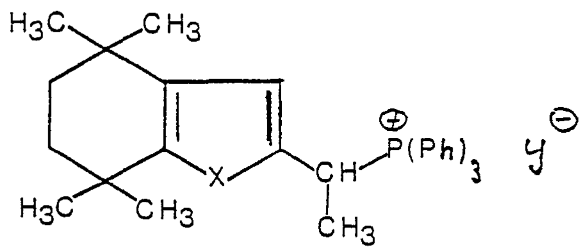

(II)

mit einer Verbindung der allgemeinen Formel

$$O = \overset{H}{\overset{|}{C}} - R^{11} \qquad \text{(III)}$$

oder eine Verbindung der allgemeinen Formel

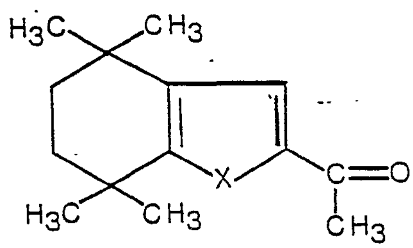

(IV)

mit einer Verbindung der allgemeinen Formel

$$(RO)_2\overset{O}{\overset{\uparrow}{P}} - CH_2R^{11} \qquad \text{(V)}$$

wobei in den obigen Formeln II-V R $C_1$-$C_6$-Alkoxy ; Ph Phenyl ; und $R^{11}$ einen Rest —$ArCO_2R^3$, —$ArSO_2$—$C_1$-$C_6$-alkyl oder —CH=CH—C($CH_3$)=$CHCO_2R^3$ darstellen und $R^1$, $R^3$, Ar und X die früher angegebene Bedeutung haben, und $Y^{\ominus}$ das Anion einer anorganischen oder organischen Säure ist, umsetzt, und gewünschtenfalls einen erhaltenen Carbonsäureester verseift, in ein Amid überführt, oder

zum Alkohol reduziert und diesen gewünschtenfalls veräthert oder zur Formylgruppe oxidiert oder eine erhaltene Carbonsäure in ein Salz überführt.

Von den anorganischen Säureanionen $Y^{\ominus}$ ist das Chlor- und Brom-ion oder das Hydrosulfat-ion, von den organischen Säureanionen ist das Tosyloxy-ion bevorzugt.

Die Umsetzung einer Formylverbindung der Formel III mit einem Phosphoniumsalz der Formel II wird in an sich bekannter Weise in Gegenwart eines säurebindenden Mittels, z. B. in Gegenwart einer starken Base, wie z. B. Butyllithium, Natriumhydrid oder dem Natriumsalz von Dimethylsulfoxyd, gegebenenfalls in einem Lösungsmittel, z. B. in einem Aether, wie Diäthyläther oder Tetrahydrofuran, oder in einem aromatischen Kohlenwasserstoff, wie Benzol in einem zwischen der Raumtemperatur und dem Siedepunkt des Reaktionsgemisches liegenden Temperaturbereich durchgeführt.

Die Umsetzung eines Phosphonats der Formel V mit einer Verbindung der Formel IV wird ebenfalls in an sich bekannter Weise in Gegenwart einer Base und, vorzugsweise, in Gegenwart eines inerten organischen Lösungsmittels, z. B. in Gegenwart von Natriumhydrid in Benzol, Toluol, Dimethylformamid, Tetrahydrofuran, Dioxan oder 1,2-Dimethoxyäthan, oder in Gegenwart eines Natriumalkoholats in einem Alkanol, z. B. Natriummethylat in Methanol, in einem zwischen 0° und dem Siedepunkt des Reaktionsgemisches liegenden Temperaturbereich durchgeführt.

Ein Carbonsäureester der Formel I kann in an sich bekannter Weise, z. B. durch Behandeln mit Alkalien, insbesondere durch Behandeln mit wässriger alkoholischer Natron- oder Kalilauge in einem zwischen der Raumtemperatur und dem Siedepunkt des Reaktionsgemisches liegenden Temperaturbereich hydrolysiert und entweder über ein Säurehalogenid oder, wie nachstehend beschrieben, unmittelbar amidiert werden.

Eine Carbonsäure der Formel I kann in an sich bekannter Weise, z. B. durch Behandeln mit Thionylchlorid, vorzugsweise in Pyridin, oder Phosphortrichlorid in Toluol in das Säurechlorid übergeführt werden, das durch Umsetzen mit Alkoholen in Ester, mit Aminen in das entsprechende Amid umgewandelt werden kann.

Ein Carbonsäureester der Formel I kann z. B. durch Behandeln mit Lithiumamid direkt in das entsprechende Amid umgewandelt werden. Das Lithiumamid wird vorteilhaft bei Raumtemperatur mit dem betreffenden Ester zur Reaktion gebracht.

Eine Carbonsäure oder ein Carbonsäureester der Formel I kann in an sich bekannter Weise zu dem entsprechenden Alkohol der Formel I reduziert werden. Die Reduktion wird vorteilhaft mit Hilfe eines Metallhydrids oder Alkylmetallhydrids in einem inerten Lösungsmittel durchgeführt. Als Hydride haben sich vor allem gemischte Metallhydride, wie Lithiumaluminiumhydrid oder bis-[Methoxy-äthylenoxy]-natriumaluminiumhydrid als geeignet erwiesen. Als Lösungsmittel verwendbar sind u. a. Aether, Tetrahydrofuran oder Dioxan, wenn Lithiumaluminiumhydrid verwendet wird ; und Aether, Hexan, Benzol oder Toluol, wenn Diisobutylaluminiumhydrid oder bis-[Methoxy-äthylenoxy]-natriumaluminiumhydrid eingesetzt werden.

Ein Alkohol der Formel I ($R^2$=$CH_2OH$) kann z. B. in Gegenwart einer Base, vorzugsweise in Gegenwart von Natriumhydrid, in einem organischen Lösungsmittel wie Dioxan, Tetrahydrofuran, 1,2-Dimethoxyäthan, Dimethylformamid, oder auch in Gegenwart eines Alkalimetallalkoholates in einem Alkanol, in einem zwischen 0° und der Raumtemperatur liegenden Temperaturbereich mit einem Alkylhalogenid, z. B. mit Aethyljodid, veräthert werden.

Ein Alkohol der Formel I kann in an sich bekannter Weise durch Behandlung mit Oxidationsmitteln wie $MnO_2$ in einem inerten Lösungsmittel, z. B. Methylenchlorid, Hexan oder Tetrahydrofuran, zu der entsprechenden Formylverbindung ($R^2$=CHO) oxidiert werden. Die Oxidation wird zweckmässig bei Raumtemperatur vorgenommen. Beispiele anderer Oxidationsmittel sind Pyridiniumchlorochromat, die Oxidation nach Pfitzner-Moffat oder der Oppenauer-Oxidation.

Eine Carbonsäure der Formel I bildet mit Basen, insbesondere mit den Alkalimetallhydroxyden, vorzugsweise mit Natrium- oder Kaliumhydroxyd Salze, die ebenfalls Gegenstand der Erfindung sind.

Die Verbindungen der Formel I können als cis/trans Gemische anfallen, welche in an sich bekannter Weise erwünschtenfalls in die cis und trans-Komponenten aufgetrennt oder zu den all trans-Verbindungen isomerisiert werden können.

Die Verbindungen der Formel I und ihre Salze können als Rodentizide verwendet werden. Sie zeigen bei Ratten bereits in geringen Dosen einen stark toxischen Effekt, der indessen erst einige Tage nach der Intoxikation erkennbar wird. Beispielsweise führte die orale Verabreichung des Aethyl-p-[2-(4,5,6,7-tetrahydro-4,4,7,7-tetramethylbenzo-[b]thien-2-yl)propenyl]benzoats an Ratten in Dosen von 1,25 mg/kg bei oraler Applikation innerhalb von 8 Tagen zum Tod aller Tiere, wobei in den ersten 5 Tagen kein Tier einging.

Von besonderem Interesse sind die Verbindungen der Formel I, in denen X=—S— oder —O—, $R^1$ ein Rest Ar—$R^2$, Ar = Phenyl oder Pyridyl und $R^2$ nieder-Alkoxycarbonyl ist, wie das Aethyl p-[2-(4,5,6,7-tetrahydro-4,4,7,7-tetramethylbenzo[b]thien-2-yl)propenyl]benzoat und der p-[2-(4,5,6,7-Tetrahydro-4,4,7,7-tetramethyl-2-benzofuranyl)-propenyl]benzoesäure-äthylester.

Die Verbindungen der Formel I können in flüssigen oder festen Ködern angewandt werden. Hierfür können alle in Rodentiziden üblichen Trägerstoffe Verwendung finden. Beispiele von Trägerstoffen sind Nahrungsmittel wie Zucker und zuckerhaltige Stoffe, Stärke, Getreide, Gelatine, Fette ; sowie mineralische Stoffe wie Calciumsilikat, Calciumcarbonat, Calciumphosphat, $SiO_2$.

Die Verbindungen der Formel I können auch in Form von festen oder flüssigen Konzentraten (Mastermix) vorliegen, die dann mit geeigneten Streckmitteln und/oder Futtermitteln, z. B. Weizen, Mais, Johannisbrotfrüchten, Bananen, Erdnüssen zu Ködern verarbeitet werden. Zweckmässig enthalten die Köder die Verbindungen der Formel I in Mengen von etwa 0,001-0,1 Gew.-%, vorzugsweise 0,005-0,01 Gew.-%.

Die folgenden Beispiele erläutern die Erfindung weiter.

Beispiel 1

30 g [1-(4,5,6,7-Tetrahydro-4,4,7,7-tetramethylbenzo[b]thien-2-yl)äthyl]triphenylphosphoniumbromid und 9,5 g 4-Aethoxycarbonylbenzaldehyd werden in 300 ml Butylenoxid suspendiert und 4 Stunden am Rückfluss erhitzt. Die so erhaltene Lösung wird im Wasserstrahlvakuum auf 1/3 des ursprünglichen Volumens eingeengt, auf 500 ml eines Methanol/Wasser-Gemisches (6 : 4) gegossen und mehrfach mit Hexan extrahiert. Die organische Phase wird dreimal mit Wasser gewaschen, über Natriumsulfat getrocknet und eingedampft. Man erhält ein leicht bräunliches Oel, das durch Filtration über Kieselgel (Eluierungsmittel Hexan/Aether 19 : 1) weiter gereinigt wird. Nach dem Umkristallisieren aus Hexan erhält man 11,3 g Aethyl p-[2-(4,5,6,7-tetrahydro-4,4,7,7-tetramethylbenzo[b]thien-2-yl)propenyl]benzoat in farblosen Kristallen, Schmelzpunkt 118-120 °C.

Das als Ausgangsprodukt verwendete [1-(4,5,6,7-Tetrahydro-4,4,7,7-tetramethylbenzo[b]thien-2-yl)äthyl]triphenylphosphoniumbromid lässt sich wie folgt herstellen :

90 g 2,5-Dimethyl-2,5-dichlorhexan und 195 ml Thiophen werden in 400 ml Hexan gelöst. Unter Eiskühlung tropft man langsam 54 ml Titantetrachlorid hinzu, erwärmt auf 40 °C während 1,5 Stunden, kühlt erneut mit Eis und versetzt das dunkelrote Reaktionsgemisch vorsichtig mit Eiswasser. Man extrahiert dreimal mit Aether, wäscht mit gesättigter Natriumbicarbonatlösung, trocknet und dampft ein. Das so erhaltene schwarze Oel wird zunächst über Kieselgel (Eluierungsmittel Hexan) filtriert und anschliessend im Wasserstrahlvakuum destilliert. Man erhält 18,5 g 4,5,6,7-Tetrahydro-4,4,7,7-tetramethylbenzo[b]thiophen als farblose Flüssigkeit, b. p. 95-96 °C/10 mm.

18,2 g 4,5,6,7-Tetrahydro-4,4,7,7-tetramethylbenzo[b]-thiophen und 7,4 g Acetylchlorid werden in 200 ml Benzol gelöst und auf 0 °C gekühlt. Bei dieser Temperatur tropft man langsam 24,4 g Zinntetrachlorid hinzu. Nach 2,5-stündigem Rühren bei Raumtemperatur kühlt man erneut auf 0 °C und tropft ein Gemisch von 9,3 ml konzentrierter Salzsäure und 36,4 ml Wasser hinzu. Das Reaktionsgemisch wird mit Aether extrahiert, einmal mit Wasser gewaschen, getrocknet, eingedampft und im Hochvakuum destilliert. Man erhält 21,7 g 2-Acetyl-4,5,6,7-tetrahydro-4,4,7,7-tetramethyl-benzo[b]thiophen als farblose Flüssigkeit, b. p. 108-115 °C/0,05 mm, die sich aus Hexan kristallisieren lässt, m. p. 53-55 °C.

21,7 g 2-Acetyl-4,5,6,7-tetrahydro-4,4,7,7-tetramethylbenzo[b]thiophen werden in 250 ml Aethanol gelöst und bei 0 °C nach und nach mit 5,5 g Natriumborhydrid versetzt. Man lässt auf Raumtemperatur kommen und rührt noch 2 Stunden. Das Reaktionsgemisch wird auf Eis gegossen, mit Aether extrahiert, einmal mit gesättigter Kochsalzlösung gewaschen, getrocknet und eingedampft. Das Rohprodukt wird über Kieselgel (Eluierungsmittel Hexan/Aether 3 : 1) filtriert und aus Hexan umkristallisiert. Man erhält 20,2 g 4,5,6,7-Tetrahydro-α,4,4,7,7-pentamethylbenzo[b]thiophen-methanol in farblosen Kristallen, m. p. 53-55 °C.

20,2 g 4,5,6,7-Tetrahydro-α,4,4,7,7-pentamethylbenzo[b]thiophen-methanol werden in 260 ml Acetonitril gelöst und mit 29,2 g Triphenylphosphoniumbromid versetzt. Nach 3-stündigem Rühren bei 50 °C dampft man zur Trockene ein, nimmt den Rückstand mit 80 % wässrigem Aethanol auf und extrahiert zweimal mit Hexan. Die Aethanolphase wird eingedampft, in Methylenchlorid gelöst, über Natriumsulfat getrocknet und eingedampft. Man erhält 49,4 g [1-(4,5,6,7-Tetrahydro-4,4,7,7-tetramethylbenzo[b]thien-2-yl)äthyl]-triphenylphosphoniumbromid als amorphes, weisses Pulver.

Beispiel 2

4,50 g [1-(4,5,6,7-Tetrahydro-4,4,7,7-tetramethyl-benzo[b]thien-2-yl)äthyl]triphenylphosphoniumbromid und 1,3 g 5-Formyl-3-methylpenta-2,4-diensäureäthylester werden in 70 ml Butylenoxid gelöst und 2 Stunden am Rückfluss erhitzt. Das abgekühlte Reaktionsgemisch wird auf ein Methanol/Wasser-Gemisch (6 : 4) gegossen und mehrfach mit Hexan extrahiert. Die Hexanphase wird dreimal mit Wasser gewaschen, getrocknet und eingedampft. Nach Filtration des Rohproduktes über Kieselgel (Eluierungsmittel Hexan/Aether 9 : 1) erhält man 2,4 g 3-Methyl-7-(4,5,6,7-tetrahydro-4,4,7,7-tramethylbenzo[b]thien-2-yl)-2,4,6-octatriensäureäthylester als schwach gelbliches Oel.

2,4 g des so erhaltenen Aethylesters werden in 40 ml Aethanol gelöst und mit einer Lösung von 2,5 g Kaliumhydroxid in 10 ml Wasser versetzt. Das Reaktionsgemisch wird 3 Stunden auf 50 °C erwärmt, auf Eiswasser gegossen, mit 2N Salzsäure angesäuert und mehrfach mit Essigester extrahiert. Die organische Phase wird mit Wasser gewaschen, getrocknet und eingedampft. Umkristallisation des Rückstandes aus Essigester ergibt 1,5 g 3-Methyl-7-(4,5,6,7-tetrahydro-4,4,7,7-tetramethylbenzo[b]thien-2-yl)-2,4,6-octatriensäure als schwach gelbliche Kristalle, m. p. 226-228 °C.

Beispiel 3

In der oben beschriebenen Weise erhält man aus 5,0 g [1-(4,5,6,7-Tetrahydro-4,4,7,7-tetramethylbenzo[b]thien-2-yl)äthyl]triphenylphosphoniumbromid und 1,65 g 5-Formyl-thiophen-2-carbonsäure-äthylester nach Umkristallisation aus Hexan 2,5 g 5-[2-(4,5,6,7-Tetrahydro-4,4,7,7-tetramethylbenzo[b]thien-2-yl)propenyl]-2-thiophencarbonsäure-äthylester in gelben Kristallen, m. p. 125-127 °C.

Beispiel 4

In der gleichen Weise erhält man aus 8,0 g [1-(4,5,6,7-Tetrahydro-4,4,7,7-tetramethylbenzo[b]thien-2-yl)äthyl]triphenylphosphoniumbromid und 2,4 g 5-Formyl-furan-2-carbonsäure-äthylester nach Umkristallisation aus Hexan 2,9 g 5-[2-(4,5,6,7-Tetrahydro-4,4,7,7-tetramethylbenzo[b]thien-2-yl)propenyl]-2-furancarbonsäure-äthylester als schwach gelbe Kristalle, m. p. 78-82 °C.

Beispiel 5

In der gleichen Weise erhält man aus 10 g [1-(4,5,6,7-Tetrahydro-4,4,7,7-tetramethylbenzo[b]thien-2-yl)äthyl]triphenylphosphoniumbromid und 3,5 g 4-Aethylsulfonylbenzaldehyd nach Umkristallisation aus Hexan/Essigester 4,5 g 2-[2-[p-(Aethylsulfonyl)phenyl]-1-methylvinyl]-4,5,6,7-tetrahydro-4,4,7,7-tetramethylbenzo[b]thiophen als schwach gelbe Kristalle, m. p. 148-150 °C.

Beispiel 6

In der gleichen Weise erhält man aus 3,9 g Triphenyl [1-(5,6,7,8-tetrahydro-5,5,8,8-tetramethyl-2-naphthyl)äthyl]phosphoniumbromid und 1,3 g 5-Formyl-thiophen-2-carbonsäure-äthylester nach Umkristallisation aus Hexan 1,8 g 5-[(E)-2-(5,6,7,8-Tetrahydro-5,5,8,8-tetramethyl-2-naphthyl)propenyl]-2-thiophencarbonsäure-äthylester als farblose Kristalle, m. p. 110-111 °C.

Beispiel 7

In der gleichen Weise erhält man aus 2,9 g Triphenyl [1-(5,6,7,8-tetrahydro-5,5,8,8-tetramethyl-2-naphthyl)äthyl]phosphoniumbromid und 0,9 g 5-Formyl-furan-2-carbonsäure-äthylester nach Umkristallisation aus Hexan 0,9 g 5-[2-(5,6,7,8-Tetrahydro-5,5,8,8-tetramethyl-2-naphthyl)-propenyl]-2-furancarbonsäure-äthylester als schwach gelbe Kristalle, m. p. 114-115 °C.

Beispiel 8

In der gleichen Weise erhält man aus 5,6 g Triphenyl [1-(5,6,7,8-tetrahydro-5,5,8,8-tetramethyl-2-naphthyl)äthyl]phosphoniumbromid und 1,4 g 6-Formyl-pyridin-3-carbonsäure-äthylester nach Chromatographie an Kieselgel (Eluierungsmittel Hexan/Aether 2 : 1) und Kristallisation aus Hexan 0,6 g 6-[(E)-2-(5,6,7,8-Tetrahydro-5,5,8-tetramethyl-2-naphthyl)propenyl]nicotinsäure-äthylester in farblosen Kristallen, m. p. 114-115 °C.

Beispiel 9

In der gleichen Weise erhält man aus 4,6 g [1-(4,5,6,7-Tetrahydro-4,4,7,7-tetramethylbenzo[b]furan-2-yl)äthyl]triphenylphosphoniumbromid und 1,5 g 4-Aethoxycarbonylbenzaldehyd nach Chromatographie an Kieselgel (Eluierungsmittel Hexan/Aether 9 : 1) und Kristallisation aus Hexan 2,2 g p-[2-(4,5,6,7-Tetrahydro-4,4,7,7-tetramethyl-2-benzofuranyl)propenyl]benzoesäure-äthylester in farblosen Kristallen, m. p. 96-97 °C.

Das als Ausgangsprodukt verwendete [1-(4,5,6,7-Tetrahydro-4,4,7,7-tetramethylbenzo[b]furan-2-yl)äthyl]triphenylphosphoniumbromid lässt sich wie folgt herstellen :

5,2 g Magnesiumspäne werden in 100 ml abs. Aether suspendiert und tropfenweise mit 13,5 ml Methyljodid versetzt. Nach Zugabe von 100 ml Aether kocht man weitere 3 Stunden am Rückfluss, bis alles Magnesium gelöst ist. Das Reaktionsgemisch wird auf 0 °C gekühlt und mit 20,7 g fein pulverisiertem Kupfer(I)jodid versetzt. Nach 15-minütigem Rühren bei 0 °C tropft man eine Lösung von 10 g 3,6,6-Trimethyl-2-cyclohexenon in 40 ml Aether hinzu und rührt weitere 4 Stunden bei 0 °C. Danach giesst man die gelbe Suspension auf Eis/2N Salzsäure, extrahiert mehrfach mit Aether, trocknet und dampft ein. Der Rückstand wird im Wasserstrahlvakuum destilliert. Man erhält 9 g 2,2,5,5-Tetramethylcyclohexanon als farblose Flüssigkeit, b. p. 71-74 °C/15 mm.

Eine Lösung von 26,4 g 2,2,5,5-Tetramethylcyclohexanon in 250 ml Aether wird bei — 20 °C zu 102,8 ml Methyllithium (2 molar in Aether) hinzugetropft. Nach 2,5-stündigem Rühren bei 0 °C giesst man auf Eis/1N Salzsäure und extrahiert mit Aether. Die organische Phase wird mit Wasser gewaschen, getrocknet und eingedampft. Der Rückstand wird im Wasserstrahlvakuum destilliert. Man erhält 22,6 g 1,2,2,5,5-Pentamethylcyclohexanol als farblose Flüssigkeit, b. p. 81-87 °C/17 mm.

22,6 g 1,2,2,5,5-Pentamethylcyclohexanol werden in 280 ml Benzol gelöst und nach Zugabe von 100 mg p-Toluolsulfonsäure 7 Stunden am Wasserabscheider gekocht. Nach dem Abkühlen der Reaktionslösung gibt man etwas festes Natriumcarbonat hinzu, filtriert und dampft bei Normaldruck ein. Der Rückstand wird im Wasserstrahlvakuum destilliert. Man erhält 15,8 g 1,3,3,6,6-Pentamethylcyclohexen als farblose Flüssigkeit, b. p. 56-57 °C/17 mm.

15 g 1,3,3,6,6-Pentamethylcyclohexen werden in 350 ml Tetrachlorkohlenstoff gelöst und mit 19,3 g n-Bromsuccinimid versetzt. Nach Zugabe einer Spatelspitze α.α'-Azoisobutyronitril kocht man 1,5 Stunden am Rückfluss. Das Reaktionsgemisch wird abgekühlt, das entstandene Succinimid abfiltriert und das Filtrat eingedampft. Man erhält 23,8 g eines schwach gelben Oels, das sofort weiterverarbeitet wird. Das Oel wird in 350 ml Dimethylformamid gelöst und mit 21 g Natrium-Benzolsulfinat versetzt. Nach 72-stündigem Rühren bei Raumtemperatur, gibt man 600 ml Aether hinzu, lässt 15 Minuten rühren, filtriert das ausgefallene Natriumbromid ab und dampft das Filtrat ein. Der Rückstand wird mit Wasser aufgenommen und mit Essigester extrahiert. Nach dem Trocknen, Eindampfen und Umkristallisieren aus Aether/Hexan erhält man 22,2 g Phenyl (3,3,6,6-tetramethyl-1-cyclohexen-1-yl)methylsulfon in weissen Kristallen, m. p. 48-50 °C.

21,6 g Phenyl (3,3,6,6-tetramethyl-1-cyclohexen-1-yl)-methylsulfon werden in 600 ml Tetrahydrofuran gelöst. Bei —40 °C tropft man 41,5 ml Butyllithium (2 molar in Hexan) hinzu und rührt noch 40 Minuten bei —40 °C. Die orange Lösung wird schnell auf eine Mischung von festem Kohlendioxid und Aether gegossen. Nach 30-minütigem Rühren versetzt man mit Wasser, säuert mit 1N Schwefelsäure an und extrahiert mit Essigester. Nach dem Trocknen, Eindampfen und Umkristallisieren aus Aether/Hexan erhält man 19,9 g 3,3,6,6-Tetramethyl-α-(phenylsulfonyl)-1-cyclohexen-1-essigsäure in farblosen Kristallen, m. p. 169-174 °C.

20,6 g 3,3,6,6-Tetramethyl-α-(phenylsulfonyl)-1-cyclohexen-1-essigsäure werden in 800 ml Aethanol gelöst und bei 0 °C mit 107,1 g Natriumamalgam (5 %) versetzt. Man rührt 4,5 Stunden bei Raumtemperatur, dekantiert vom Quecksilber, säuert mit 2N Salzsäure an und extrahiert mit Essigester. Die organische Phase wird mit Wasser gewaschen, getrocknet, eingedampft und über Kieselgel (Eluierungsmittel Hexan/Aether 1 : 1) filtriert. Nach dem Umkristallisieren aus Hexan erhält man 10,2 g 3,3,6,6-Tetramethyl-1-cyclohexen-1-essigsäure in farblosen Kristallen, m. p. 50-54 °C.

10,2 g 3,3,6,6-Tetramethyl-1-cyclohexen-1-essigsäure werden in 140 ml Methylenchlorid gelöst und man gibt bei Raumtemperatur eine Lösung von 14,2 g Thallium(I)äthylat in 80 ml Methylenchlorid hinzu. Nun kühlt man die milchigweisse Lösung auf 0 °C und tropft langsam eine Lösung von 2,7 ml Brom in 70 ml Methylenchlorid hinzu. Nach 4-stündigem Rühren bei Raumtemperatur giesst man auf Eis/Wasser und extrahiert mit Methylenchlorid. Die organische Phase wird mit Wasser gewaschen, getrocknet und eingedampft. Das so erhaltene Rohprodukt wird über Kieselgel (Eluierungsmittel Hexan/Aether 4 : 1) filtriert. Man erhält 6,5 g des entsprechenden Lactons, das sofort weiterverarbeitet wird. 6,5 g Lacton werden in 200 ml Methylenchlorid gelöst und bei —70 °C tropfenweise mit 40 ml Diisobutylaluminiumhydrid (20 % in Toluol) versetzt. Nach 3,5-stündigem Rühren bei —70 °C tropft man 200 ml eines Methanol/Wasser-Gemisches (1 : 1) hinzu, lässt auf 0 °C kommen und tropft weitere 100 ml Wasser hinzu. Nun wird mit 1N Salzsäure angesäuert und mit Aether extrahiert. Die organische Phase wird mit Wasser gewaschen, getrocknet und eingedampft. Nach Chromatographie des Rohproduktes an Kieselgel (Eluierungsmittel Hexan/Aether 9 : 1) erhält man 4,8 g 4,5,6,7-Tetrahydro-4,4,7,7-tetramethyl-benzo[b]furan als farblose Flüssigkeit.

1,6 g 4,5,6,7-Tetrahydro-4,4,7,7-tetramethyl-benzo[b]furan werden in 40 ml Tetrahydrofuran gelöst und bei —30 °C tropfenweise mit 5,2 ml Butyllithium (2 normal in Hexan) versetzt. Nach 3,5-stündigem Rühren bei —30 °C tropft man eine Lösung von 550 mg Acetaldehyd in 10 ml Tetrahydrofuran dazu. Man lässt auf Raumtemperatur kommen, giesst das Reaktionsgemisch auf Eis, extrahiert mit Aether, trocknet und dampft ein. Das so erhaltene, schwach gelbliche Oel (2,2 g) wird in 35 ml Acetonitril gelöst und mit 3,8 g Triphenylphosphoniumbromid versetzt. Man erwärmt 3 Stunden auf 50 °C und dampft anschliessend zur Trockene ein. Der ölige Rückstand wird in 80 % wässrigem Aethanol gelöst, zweimal mit Hexan extrahiert und die Aethanolphase zur Trockene eingedampft. Der Rückstand wird in Methylenchlorid gelöst, über Natriumsulfat getrocknet und eingedampft. Man erhält 4,6 g [1-(4,5,6,7-Tetrahydro-4,4,7,7-tetramethylbenzo[b]furan-2-yl)äthyl]triphenylphosphoniumbromid als farblose, amorphe Substanz.

## Beispiel 10

3,0 g Aethyl p-[2-(4,5,6,7-tetrahydro-4,4,7,7-tetramethylbenzo[b]thien-2-yl)propenyl]benzoat werden in 100 ml Aethanol gelöst und mit einer Lösung von 3,9 g Kaliumhydroxid in 20 ml Wasser versetzt. Nach 3-stündigem Rühren bei 50 °C kühlt man ab, giesst auf Eiswasser und säuert mit 2N Schwefelsäure an. Das Reaktionsgemisch wird mit Essigester extrahiert, die organische Phase mit Wasser gewaschen, über Natriumsulfat getrocknet und eingedampft. Nach dem Umkristallisieren des Rückstandes aus Essigester erhält man 2,4 g p-[(E)-2-(4,5,6,7-Tetrahydro-4,4,7,7-tetramethylbenzo[b]thien-2-yl)propenyl]benzoesäure in schwach gelblichen Kristallen, m. p. 236-238 °C.

## Beispiel 11

10,2 g [1-(4,5,6,7-Tetrahydro-4,4,7,7-tetramethylbenzo[b]thien-2-yl)äthyl]triphenylphosphoniumbromid und 2,5 g 6-Formyl-pyridin-3-carbonsäure-äthylester werden in 100 ml Butylenoxid suspendiert und 1,5 Stunden am Rückfluss erhitzt. Nach der in Beispiel 1 beschriebenen Aufarbeitung erhält man 6,9 g eines gelben Oeles, das zur Abtrennung des Z-Isomeren über Kieselgel (Eluierungsmittel Hexan/Aether = 9 : 1) chromatographiert wird. Das langsamer laufende E-Isomere wird aus Hexan umkristallisiert. Man erhält 1,3 g 6-[(E)-2-(4,5,6,7-Tetrahydro-4,4,7,7-tetramethylbenzo[b]thien-2-yl)propenyl]nicotinsäure-äthylester in gelblichen Kristallen, m. p. 83-84 °C.

## Beispiel 12

Ein rodentizides Mittel kann die folgende Zusammensetzung aufweisen :

A.

| | |
|---|---|
| Wirkstoff, z. B. Aethyl p-[2-(4,5,6,7-tetrahydro-4,4,7,7-tetramethylo-[b]thien-2-yl)propenyl]benzoat | 0,005 % |
| Konservierungsmittel (z. B. chlorierte Phenole) | 0,500 % |
| Hydrophobiermittel (z. B. natürliche und synthetische Wachse) | 5,000 % |
| Farbstoff (z. B. Berlinerblau) | 0,300 % |
| Lockmittel (z. B. Eiweisshydrolysat) | 2,500 % |
| Feuchthaltemittel (z. B. Polyglykole) | 0,460 % |
| Neutralisationsmittel (z. B. Alkylolamine) | 0,060 % |
| Trägerstoff (anorganisch) (z. B. Kreide) | 4,500 % |
| Trägerstoff (organisch) (z. B. Weizenmehl) | 86,675 % |
| | 100,000 % |

B.

| | |
|---|---|
| variable Mengen Wirkstoff (z. B. 0,005 %) | |
| Maismehl | 65 % |
| Haferflocken | 25 % |
| Maisöl | 5 % |
| Zucker | ad 100 % |

## Beispiel 13

Mastermixes können folgende Zusammensetzung aufweisen :

A.
Flüssiger Mastermix

| | |
|---|---|
| Wirkstoff | 0,25 % |
| Oel, z. B. Pflanzensamenöl oder Mineralöl | 99,75 % |
| | 100,00 % |

B. Fester Mastermix

| | |
|---|---|
| Wirkstoff | 0,1 % |
| Stäubeverhinderungsmittel, z. B. Mineralöl | 10,0 % |
| Anorganischer Träger, z. B. Kaolin | 89,9 % |
| | 100,0 % |

Diese Mastermixes werden mit verschiedenen Ködern (z. B. Weizen, Mais, Johannisbrotfrüchten, Bananen, Erdnüsse, etc.) so vermengt, dass Wirkstoffkonzentrationen von z. B. 0,005 % entstehen.

**Patentansprüche** (für die Vertragsstaaten : BE, CH, DE, FR, GB, IT, LI, LU, NL, SE)

1. Verbindungen der allgemeinen Formel

(Siehe Formel Seite 8 f.)

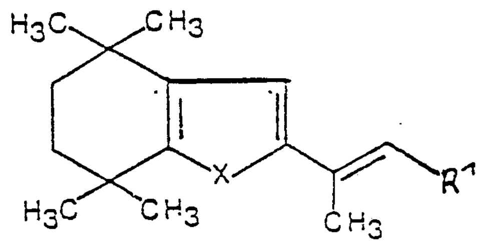

(I)

worin X —CH=CH—, —O— oder —S— ist ; $R^1$ ein Rest Ar—$R^2$ oder —CH=CH—C($CH_3$)=CH—$R^{21}$ ; Ar Phenyl, Pyridyl, Furyl oder Thienyl ; $R^2$ ein Rest —$CO_2R^3$, —C(O)$R^4$, —$CH_2OR^3$, $C_1$-$C_6$-Alkylsulfonyl oder Formyl ; $R^{21}$ ein Rest —$CO_2R^3$, —C(O)$R^4$, —$CH_2OR^3$ oder Formyl ; $R^3$ Wasserstoff oder $C_1$-$C_6$-Alkyl und $R^4$ Wasserstoff, Hydroxy, Amino, $C_1$-$C_6$-Alkylamino, di-($C_1$-$C_6$-Alkyl)amino oder $C_1$-$C_6$-Alkyl ist ; wobei mindestens ein Ring des Moleküls heterocyclisch ist, und Salze davon.

2. Verbindungen gemäss Anspruch 1, in denen X —S— oder —O— ist.

3. Verbindungen gemäss Anspruch 2, in denen $R^1$ ein Rest Ar—$R^2$ und Ar Phenyl oder Pyridyl ist.

4. Verbindungen gemäss den Ansprüchen 1-3, in denen $R^2$ $C_1$-$C_6$-Alkoxycarbonyl ist.

5. Aethyl-p-[2-(4,5,6,7-tetrahydro-4,4,7,7-tetramethylbenzo[b]thien-2-yl)propenyl]benzoat.

6. 6-[(E)-2-(4,5,6,7-Tetrahydro-4,4,7,7-tetramethylbenzo[b]thien-2-yl)propenyl]nicotinsäure-äthylester.

7. 3-Methyl-7-(4,5,6,7-tetrahydro-4,4,7,7-tetramethylbenzo[b]thien-2-yl)-2,4,6-octatriensäure, 5-[2-(4,5,6,7-Tetrahydro-4,4,7,7-tetramethylbenzo[b]thien-2-yl)propenyl]-2-thiophencarbonsäure-äthylester, 5-[2-(4,5,6,7-Tetrahydro-4,4,7,7-tetramethylbenzo[b]thien-2-yl)propenyl]-2-furancarbonsäure-äthylester, 2-[2-[p-(Aethylsulfonyl)phenyl]-1-methylvinyl]-4,5,6,7-tetrahydro-4,4,7,7-tetramethylbenzo-[b]thiophen, 5-[(E)-2-(5,6,7,8-Tetrahydro-5,5,8,8-tetramethyl-2-naphthyl)propenyl]-2-thiophencarbonsäure-äthylester, 5-[2-(5,6,7,8-Tetrahydro-5,5,8,8-tetramethyl-2-naphthyl)-propenyl]-2-furancarbonsäure-äthylester, 6-[(E)-2-(5,6,7,8-Tetrahydro-5,5,8,8-tetramethyl-2-naphthyl)propenyl]nicotin-säure-äthylester, p-[2-(4,5,6,7-Tetrahydro-4,4,7,7-tetramethyl-2-benzofuranyl)propenyl]benzoesäure-äthylester und p-[(E)-2-(4,5,6,7-Tetrahydro-4,4,7,7-tetramethylbenzo[b]-thien-2-yl)propenyl]benzoesäure.

8. Rodentizid, enthaltend eine Verbindung der Formel I aus Anspruch 1 und übliche Trägerstoffe.

9. Verwendung von Verbindungen der Formel I aus Anspruch 1 als Rodentizid.

10. Verfahren zur Herstellung von Verbindungen der allgemeinen Formel I aus Anspruch 1, dadurch gekennzeichnet, dass man eine Verbindung der allgemeinen Formel

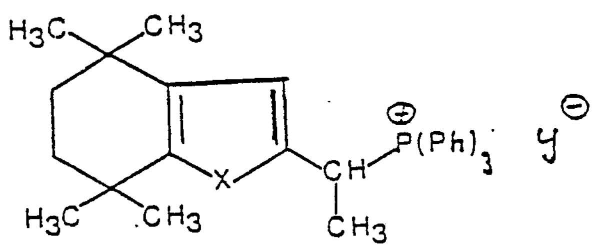

(II)

mit einer Verbindung der allgemeinen Formel

$$O = \overset{H}{\overset{|}{C}} - R^{11} \qquad (III)$$

oder eine Verbindung der allgemeinen Formel

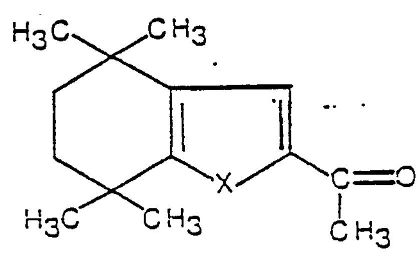

(IV)

mit einer Verbindung der allgemeinen Formel

$$(RO)_2\overset{O}{\overset{\uparrow}{P}} - CH_2R^{11} \qquad (V)$$

wobei in den obigen Formeln II-V R $C_1$-$C_6$-Alkoxy ; Ph Phenyl ; und $R^{11}$ einen Rest $—ArCO_2R^3$, $—ArSO_2—C_1$-$C_6$-alkyl oder $—CH{=}CH—C(CH_3){=}CHCO_2R^3$ darstellen und $R^1$, $R^3$, Ar und X die früher angegebene Bedeutung haben, und $Y^-$ das Anion einer anorganischen oder organischen Säure ist, umsetzt, und gewünschtenfalls einen erhaltenen Carbonsäureester verseift, in ein Amid überführt, oder zum Alkohol reduziert und diesen gewünschtenfalls veräthert oder zur Formylgruppe oxidiert oder eine erhaltene Carbonsäure in ein Salz überführt.

**Patentansprüche** (für den Vertragsstaat AT)

1. Rodentizid, enthaltend eine Verbindung der allgemeinen Formel

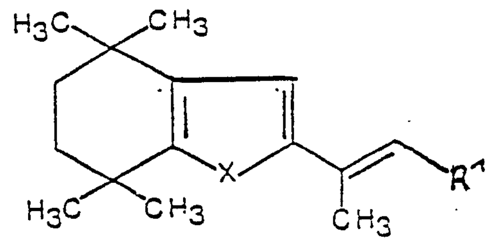

(I)

worin X $—CH{=}CH—$, $—O—$ oder $—S—$ ist ; $R^1$ ein Rest $Ar—R^2$ oder $—CH{=}CH—C(CH_3){=}CH—R^{21}$ ; Ar Phenyl, Pyridyl, Furyl oder Thienyl ; $R^2$ ein Rest $—CO_2R^3$, $—C(O)R^4$, $—CH_2OR^3$, $C_1$-$C_6$-Alkylsulfonyl oder Formyl ; $R^{21}$ ein Rest $—CO_2R^3$, $—C(O)R^4$, $—CH_2OR^3$ oder Formyl ; $R^3$ Wasserstoff oder $C_1$-$C_6$-Alkyl und $R^4$ Wasserstoff, Hydroxy, Amino, $C_1$-$C_6$-Alkylamino, di-($C_1$-$C_6$-Alkyl)amino oder $C_1$-$C_6$-Alkyl ist ; wobei mindestens ein Ring des Moleküls heterocyclisch ist,
oder ein Salz davon, und übliche Trägerstoffe.

2. Rodentizid nach Anspruch 1, wobei in der Verbindung der Formel I X $—S—$ oder $—O—$ ist.

3. Rodentizid nach Anspruch 2, wobei in der Verbindung der Formel I $R^1$ ein Rest $Ar—R^2$ und $R^2$ Phenyl oder Pyridyl ist.

4. Rodentizid nach den Ansprüchen 1-3, wobei in der Verbindung der Formel I $R^2$ $C_1$-$C_6$-Alkoxycarbonyl ist.

5. Rodentizid nach den Ansprüchen 1-4, wobei die Verbindung der Formel I Aethyl-p-[2-(4,5,6,7-tetrahydro-4,4,7,7-tetramethylbenzo[b]thien-2-yl)propenyl]benzoat ist.

6. Rodentizid nach den Ansprüchen 1-4, wobei die Verbindung der Formel I 6-[(E)-2-(4,5,6,7-Tetrahydro-4,4,7,7-tetramethylbenzo[b]thien-2-yl)propenyl]nicotinsäure-äthylester ist.

7. Verfahren zur Herstellung von Verbindungen der allgemeinen Formel

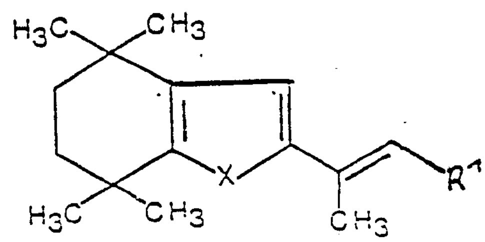

(I)

worin X $—CH{=}CH—$, $—O—$ oder $—S—$ ist ; $R^1$ ein Rest $Ar—R^2$ oder $—CH{=}CH—C(CH_3){=}CH—R^{21}$ ; Ar Phenyl, Pyridyl, Furyl oder Thienyl ; $R^2$ ein Rest $—CO_2R^3$, $—C(O)R^4$, $—CH_2OR^3$, $C_1$-$C_6$-Alkylsulfonyl oder Formyl ; $R^{21}$ ein Rest $—CO_2R^3$, $—C(O)R^4$, $—CH_2OR^3$ oder Formyl ; $R^3$ Wasserstoff oder $C_1$-$C_6$-Alkyl und $R^4$ Wasserstoff, Hydroxy, Amino, $C_1$-$C_6$-Alkylamino, di-($C_1$-$C_6$-Alkyl)amino oder $C_1$-$C_6$-Alkyl ist ; wobei mindestens ein Ring des Moleküls heterocyclisch ist,
dadurch gekennzeichnet, dass man eine Verbindung der allgemeinen Formel

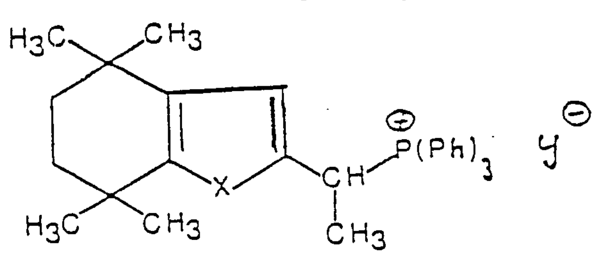

(II)

mit einer Verbindung der allgemeinen Formel

$$O = \overset{H}{\overset{|}{C}} - R^{11} \qquad (III)$$

oder eine Verbindung der allgemeinen Formel

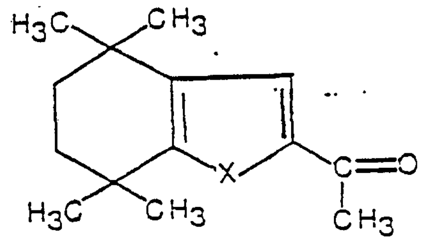

(IV)

mit einer Verbindung der allgemeinen Formel

$$(RO)_2\overset{O}{\overset{\uparrow}{P}} - CH_2R^{11} \qquad (V)$$

wobei in den obigen Formeln II-V R $C_1$-$C_6$-Alkoxy; Ph Phenyl; und $R^{11}$ einen Rest —$ArCO_2R^3$. —$ArSO_2$—$C_1$-$C_6$-alkyl oder —CH=CH—$C(CH_3)$=$CHCO_2R^3$ darstellen und $R^1$, $R^3$, Ar und X die früher angegebene Bedeutung haben, und $Y^-$ das Anion einer anorganischen oder organischen Säure ist, umsetzt, und gewünschtenfalls einen erhaltenen Carbonsäureester verseift, in ein Amid überführt, oder zum Alkohol reduziert und diesen gewünschtenfalls veräthert oder zur Formylgruppe oxidiert oder eine erhaltene Carbonsäure in ein Salz überführt.

8. Verfahren zur Herstellung eines Rodentizids, dadurch gekennzeichnet, dass man eine Verbindung der Formel I aus Anspruch 1 oder ein Salz davon mit üblichen Trägerstoffen umsetzt.

9. Verwendung von Verbindungen der Formel I aus Anspruch 1 zur Bekämpfung von schädlichen Nagetieren.

**Claims** (for the Contracting States: BE, CH, DE, FR, GB, IT, LI, LU, NL, SE)

1. Compounds of the general formula

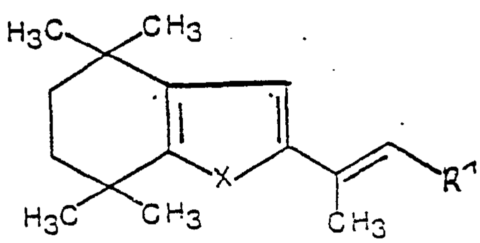

(I)

wherein X is —CH=CH—, —O— or —S—; $R^1$ is a residue Ar—$R^2$ or —CH=CH—$C(CH_3)$=CH—$R^{21}$; Ar is phenyl, pyridyl, furyl or thienyl; $R^2$ is a residue —$CO_2R^3$, —$C(O)R^4$, —$CH_2OR^3$, $C_1$-$C_6$-alkyl-sulphonyl or formyl; $R^{21}$ is a residue —$CO_2R^3$, —$C(O)R^4$, —$CH_2OR^3$ or formyl; $R^3$ is hydrogen or $C_1$-$C_6$-alkyl and $R^4$ is hydrogen, hydroxy, amino, $C_1$-$C_6$-alkylamino, di-($C_1$-$C_6$-alkyl)amino or $C_1$-$C_6$-alkyl; whereby at least one ring of the molecule is heterocyclic,
and salts thereof.

2. Compounds in accordance with claim 1, in which S is —S— or —O—.

3. Compounds in accordance with claim 2, in which $R^1$ is a residue Ar—$R^2$ and Ar is phenyl or pyridyl.

4. Compounds in accordance with claims 1-3, in which $R^2$ is $C_1$-$C_6$-alkoxycarbonyl.

5. Ethyl p-[2-(4,5,6,7-tetrahydro-4,4,7,7-tetramethylbenzo[b]thien-2-yl)propenyl]benzoate.

6. 6-[(E)-2-(4,5,6,7-Tetrahydro-4,4,7,7-tetramethylbenzo[b]thien-2-yl)propenyl]nicotinic acid ethyl ester.

7. 3-Methyl-7-(4,5,6,7-tetrahydro-4,4,7,7-tetramethylbenzo[b]thien-2-yl)-2,4,6-octatrienoic acid, 5-[2-(4,5,6,7-tetrahydro-4,4,7,7-tetramethylbenzo[b]thien-2-yl)propenyl]-2-thiophenecarboxylic acid ethyl ester, 5-[2-(4,5,6,7-tetrahydro-4,4,7,7-tetramethylbenzo[b]thien-2-yl)propenyl]-2-furancarboxylic acid ethyl ester, 2-[2-[p-(ethylsulphonyl)phenyl]-1-methylvinyl]-4,5,6,7-tetrahydro-4,4,7,7-tetramethylbenzo[b]thiophene, 5-[(E)-2-(5,6,7,8-tetrahydro-5,5,8,8-tetramethyl-2-naphthyl)propenyl]-2-thiophenecarboxylic acid ethyl ester, 5-[2-(5,6,7,8-tetrahydro-5,5,8,8-tetramethyl-2-naphthyl)propenyl]-2-furancarboxylic acid ethyl ester, 6-[(E)-2-(5,6,7,8-tetrahydro-5,5,8,8-tetramethyl-2-naphthyl)propenyl]-nicotinic acid ethyl ester, p-[2-(4,5,6,7-tetrahydro-4,4,7,7-tetramethyl-2-benzofuranyl)propenyl]benzoic acid ethyl ester and p-[(E)-2-(4,5,6,7-tetrahydro-4,4,7,7-tetramethylbenzo[b]thien-2-yl)propenyl]benzoic acid.

8. A rodenticide containing a compound of formula I from claim 1 and customary carrier materials.

9. The use of compounds of formula I from claim 1 as rodenticides.

10. A process for the manufacture of compounds of general formula I from claim 1, characterized in that a compound of the general formula

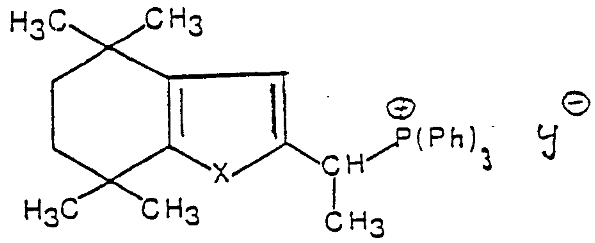

(II)

is reacted with a compound of the general formula

$$O = \overset{H}{C} - R^{11}$$ (III)

or a compound of the general formula

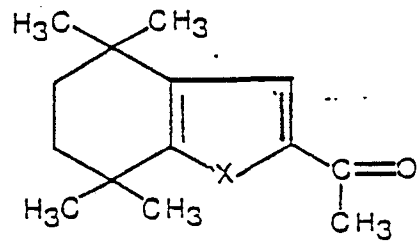

(IV)

is reacted with a compound of the general formula

$$(RO)_2\overset{O}{\overset{\uparrow}{P}} - CH_2R^{11}$$ (V)

wherein in the above formulae II-V R represents $C_1$-$C_6$-alkoxy ; Ph represents phenyl ; and $R^{11}$ represents a residue $—ArCO_2R^3$, $—ArSO_2—C_1$-$C_6$-alkyl or $—CH{=}CH—C(CH_3){=}CHCO_2R^3$ and $R^1$, $R^3$, Ar and X have the significance given earlier, and $Y^-$ is the anion of an inorganic or organic acid,

and, if desired, a carboxylic acid ester obtained is saponified, converted into an amide or reduced to the alcohol and, if desired, this is etherified or oxidized to the formyl group or a carboxylic acid obtained is converted into a salt.

**Claims** (for the Contracting State AT)

1. A rodenticide containing a compound of the general formula

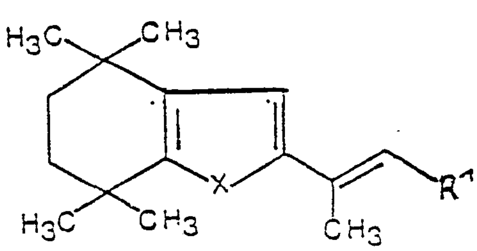

(I)

wherein X is —CH=CH—, —O— or —S— ; $R^1$ is a residue Ar—$R^2$ or —CH=CH—C($CH_3$)=CH—$R^{21}$ ; Ar is phenyl, pyridyl, furyl or thienyl ; $R^2$ is a residue —$CO_2R^3$, —C(O)$R^4$, —$CH_2OR^3$, $C_1$-$C_6$-alkyl-sulphonyl or formyl ; $R^{21}$ is a residue —$CO_2R^3$, —C(O)$R^4$, —$CH_2OR^3$ or formyl ; $R^3$ is hydrogen or $C_1$-$C_6$-alkyl and $R^4$ is hydrogen, hydroxy, amino, $C_1$-$C_6$-alkylamino, di-($C_1$-$C_6$-alkyl)amino or $C_1$-$C_6$-alkyl ; whereby at least one ring of the molecule is heterocyclic,
or a salt thereof, and customary carrier materials.

2. A rodenticide according to claim 1, in which in the compound of formula I X is —S— or —O—.

3. A rodenticide according to claim 2, wherein in the compound of formula 1 $R^1$ is a residue Ar—$R^2$ and $R^2$ is phenyl or pyridyl.

4. A rodenticide according to claims 1-3, wherein in the compound of formula I $R^2$ is $C_1$-$C_6$-alkoxycarbonyl.

5. A rodenticide according to claims 1-4, wherein the compound of formula I is ethyl p-[2-(4,5,6,7-tetrahydro-4,4,7,7-tetramethylbenzo[b]thien-2-yl)propenyl]-benzoate.

6. A rodenticide according to claims 1-4, wherein the compound of formula I is 6-[(E)-2-(4,5,6,7-tetrahydro-4,4,7,7-tetramethylbenzo[b]thien-2-yl)propenyl]nicotinic acid ethyl ester.

7. A process for the manufacture of compounds of the general formula

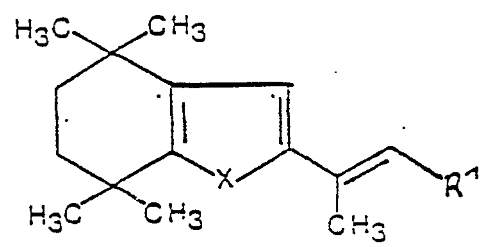

(I)

wherein X is —CH=CH—, —O— or —S— ; $R^1$ is a residue Ar—$R^2$ or —CH=CH—C($CH_3$)=CH—$R^{21}$ ; Ar is phenyl, pyridyl, furyl or thienyl ; $R^2$ is a residue —$OO_2R^3$, —C(O)$R^4$, —$CH_2OR^3$, $C_1$-$C_6$-alkyl-sulphonyl or formyl ; $R^{21}$ is a residue —$CO_2R^3$, —O(O)$R^4$, —$CH_2OR^3$ or formyl ; $R^3$ is hydrogen or $C_1$-$C_6$-alkyl and $R^4$ is hydrogen, hydroxy, amino, $C_1$-$C_6$-alkylamino, di-($C_1$-$C_6$-alkyl)amino or $C_1$-$C_6$-alkyl ; whereby at least one ring of the molecule is heterocyclic,
characterized in that a compound of the general formula

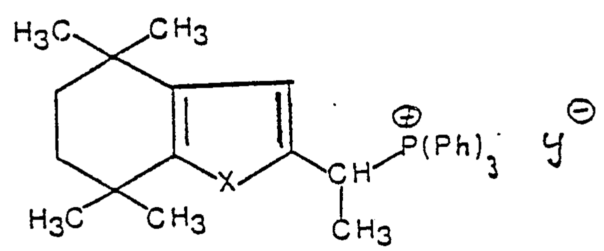

(II)

is reacted with a compound of the general formula

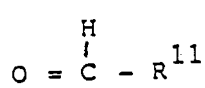

(III)

or a compound of the general formula

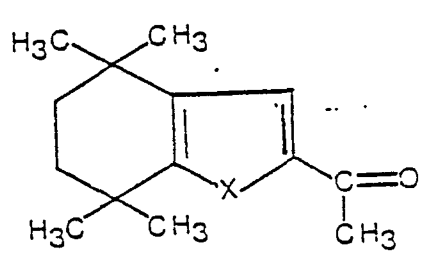

(IV)

is reacted with a compound of the general formula

$$(RO)_2\overset{\overset{O}{\uparrow}}{P} - CH_2R^{11} \qquad (V)$$

wherein in the above formulae II-V R represents $C_1$-$C_6$-alkoxy ; Ph represents phenyl ; and $R^{11}$ represents a residue $—ArCO_2R^3$, $—ArSO_2—C_1$-$C_6$-alkyl or $—CH{=}CH—C(CH_3){=}CHCO_2R^3$ and $R^1$, $R^3$, Ar and X have the significance given earlier, and $Y^-$ is the anion of an inorganic or organic acid,
and, if desired, a carboxylic acid ester obtained is saponified, converted into an amide or reduced to the alcohol and, if desired, this is etherified or oxidized to the formyl group or a carboxylic acid obtained is converted into a salt.

8. A process for the manufacture of a rodenticide, characterized in that a compound of formula I from claim 1 or a salt thereof is mixed with customary carrier materials.

9. The use of compounds of formula 1 from claim 1 for the control of harmful rodents.

**Revendications** (pour les Etats contractants : BE, CH, DE, FR, GB, IT, LI, LU, NL, SE)

1. Composés de formule générale

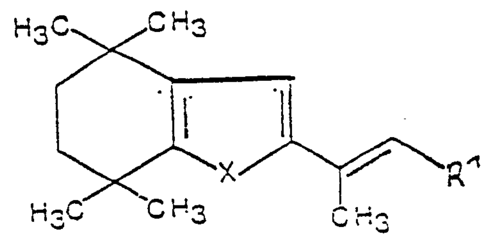

(I)

où X représente —CH=CH—, —O— ou —S—; R1 est un radical Ar—R2 ou $—CH{=}CH—C(CH_3){=}CH—R21$ ; Ar est un phényle, pyridyle, furyle ou thiényle ; R2 est un radical $—CO_2R3$, —C(O)R4, $—CH_2OR3$, alcoyle en C1 à C6-sulfonyle ou formyle ; R21 est un radical $—CO_2$ R3, —C(O)R4, $—CH_2$ OR3 ou formyle ; R3 est un hydrogène ou un alcoyle en C1 à C6 et R4 est un hydrogène, hydroxy, amino, alcoyle en C1 à C6-amino, di-(alcoyle en C1 à C6)amino ou alcoyle en C1 à C6 ; où au moins un noyau de la molécule est hétérocyclique,
et leurs sels.

2. Composés selon la revendication 1, dans lesquels X est —S— ou —O—.

3. Composés selon la revendication 2, dans lesquels R1 est un radical Ar—R2 et Ar est un phényle ou un pyridyle.

4. Composés selon les revendications 1-3, dans lesquels R2 est un alcoxycarbonyle en C1 à C6.

5. Ethyl-p-[2-(4,5,6,7-tétrahydro-4,4,7,7-tétraméthylbenzo[b]thién-2-yl)propényl]benzoate.

6. Ester éthylique de l'acide 6-[(E)-2-(4,5,6,7-tétrahydro-4,4,7,7-tétraméthylbenzo[b]thién-2-yl)propényl]nicotinique.

7. Acide 3-méthyl-7-(4,5,6,7-tétrahydro-4,4,7,7-tétraméthylbenzo[b]thién-2-yl)-2,4,6-octatriénoïque, ester éthylique de l'acide 5-[2-(4,5,6,7-tétrahydro-4,4,7,7-tétraméthylbenzo[b]thién-2-yl)propényl]-2-thiophènecarboxylique, Ester éthylique de l'acide 5-[2-(4,5,6,7-tétrahydro-4,4,7,7-tétraméthylbenzo[b]thién-2-yl)propényl]-2-furannecarboxylique, 2-[2-]p-(éthylsulfonyl)phényl[-1-méthylvinyl]-4,5,6,7-tétrahydro-4,4,7,7-tétraméthylbenzo[b]thiophène, ester éthylique de l'acide 5-[(E)-2-(5,6,7,8-tétrahydro-5,5,8,8-tétraméthyl-2-naphtyl)propényl]-2-thiophènecarboxylique, ester éthylique de l'acide 5-[2-(5,6,7,8-tétrahydro-5,5,8,8-tétraméthyl-2-naphtyl)-propényl]-2-furannecarboxylique, ester éthylique de l'acide 6-[(E)-2-(5,6,7,8-tétrahydro-5,5,8,8-tétraméthyl-2-naphtyl)-propényl]nicotinique, ester éthylique de l'acide p-[2-(4,5,6,7-tétrahydro-4,4,7,7-tétraméthyl-2-benzofuranyl)propényl]benzoïque et acide p-[(E)-2-(4,5,6,7-tétrahydro-4,4,7,7-tétraméthylbenzo[b]thién-2-yl)propényl]-benzoïque.

8. Rodenticide contenant un composé de formule I de la revendication 1 et les supports habituels.

9. Application de composés de formule I de la revendication 1 comme rodenticides.

10. Procédé de préparation de composés de formule générale I de la revendication 1, caractérisé en ce qu'on fait réagir un composé de formule générale

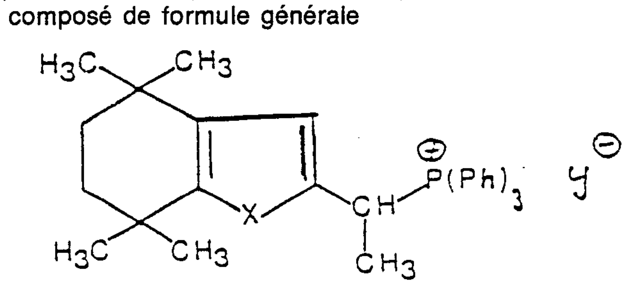

(II)

avec un composé de formule générale

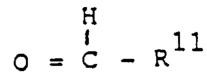

(III)

ou un composé de formule générale

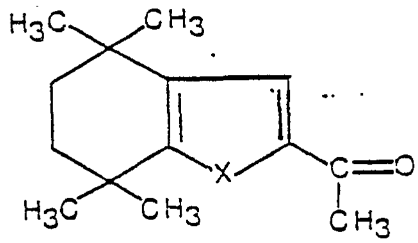

(IV)

avec un composé de formule générale

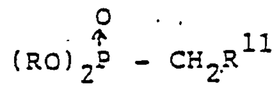

(V)

où dans les formules II-V ci-dessus R représente un alcoxy en C1 à C6 ; Ph représente un phényle : et R11 représente un radical —$ArCO_2R3$, —$ArSO_2$-alcoyle en C1 à C6 ou —CH=CH—$C(CH_3)$=$CHCO_2$ R3 et R1, R3, Ar et X ont la signification donnée ci-dessus, et $Y^-$ est l'anion d'un acide inorganique ou organique, et si on le désire en ce qu'on saponifie un ester d'acide carboxylique obtenu, en ce qu'on le transforme en un amide, ou en ce qu'on le réduit en alcool et en ce que si on le désire on éthérifie celui-ci ou on l'oxyde en groupe formyle ou on transforme un acide carboxylique obtenu en un sel.

**Revendications** (pour l'Etat contractant AT)

1. Rodenticide contenant un composé de formule générale

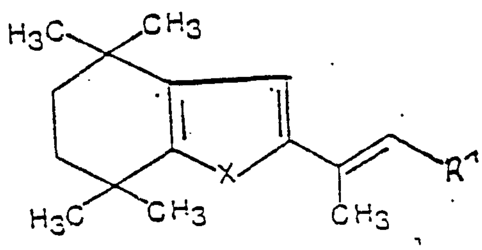

(I)

où X représente —CH=CH—, —O— ou —S— ; R1 représente un radical Ar—R2 ou —CH=CH—$C(CH_3)$=CH—R21 ; Ar représente un phényle, pyridyle, furyle ou thiényle ; R2 représente un radical —$CO_2R3$, —C(O)R4, —$CH_2OR3$, alcoyle en C1 à C6-sulfonyle ou formyle ; R21 représente un radical —$CO_2R3$, —C(O)R4, —$CH_2OR3$ ou formyle ; R3 représente un hydrogène ou un alcoyle en C1 à C6 et R4 représente un hydrogène, hydroxy, amino, alcoyle en C1 à C6-amino, di-(alcoyle en C1 à C6) amino ou alcoyle en C1 à C6 ; où au moins un noyau de la molécule est hétérocyclique,
ou un de ses sels, et les supports habituels.

2. Rodenticide selon la revendication 1, où dans le composé de formule I X représente —S— ou —O—.

3. Rodenticide selon la revendication 2, où dans le composé de formule I R1 est un radical Ar—R2 et R2 est un phényle ou un pyridyle.

4. Rodenticide selon les revendications 1-3, où dans le composé de formule I R2 est un alcoxycarbonyle en C1 à C6.

5. Rodenticide selon les revendications 1-4, où le composé de formule I est l'éthyl-p-[2-(4,5,6,7-tétrahydro-4,4,7,7-tétraméthylbenzo[b]thién-2-yl)propényl]benzoate.

6. Rodenticide selon les revendications 1-4, où le composé de formule I est l'ester éthylique de l'acide 6-[(E)-2-(4,5,6,7-tétrahydro-4,4,7,7-tétraméthylbenzo[b]thién-2-yl)propényl]nicotinique.

7. Procédé de préparation de composés de formule générale

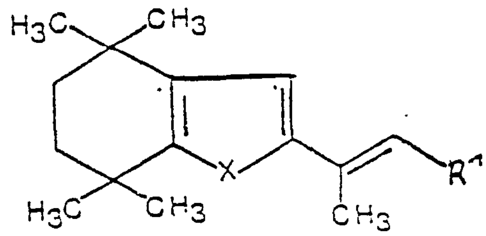

(I)

où X représente —CH=CH—, —O— ou —S— ; R1 est un radical Ar—R2 ou —CH=CH—C($CH_3$)=CH—R21 ; Ar représente un phényle, pyridyle, furyle ou thiényle ; R2 est un radical —$CO_2$R3, —C(O)R4, —$CH_2$OR3, alcoylsulfonyle en C1 à C6 ou formyle ; R21 est un radical —$CO_2$R3, —C(O)R4, —$CH_2$OR3 ou formyle ; R3 est un hydrogène ou un alcoyle en C1 à C6 et R4 est un hydrogène, hydroxy, amino, alcoylamino en C1 à C6, di-(alcoyle en C1 à C6) amino ou alcoyle en C1 à C6 ; où au moins un noyau de la molécule est hétérocyclique,
caractérisé en ce qu'on fait réagir un composé de formule générale

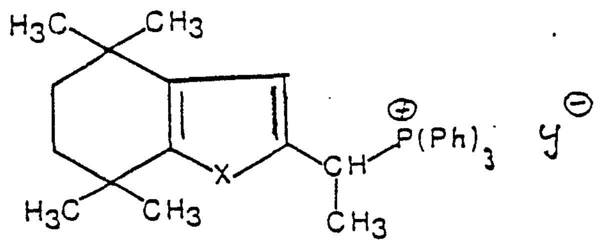

(II)

avec un composé de formule générale

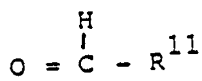

(III)

ou un composé de formule générale

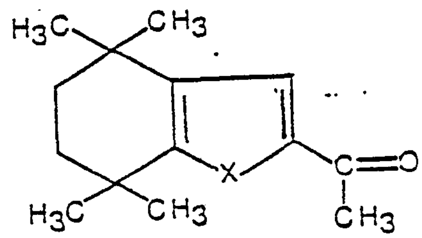

(IV)

avec un composé de formule générale

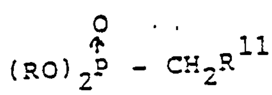

(V)

où dans les formules II-V ci-dessus R représente un alcoxy en C1 à C6 ; Ph un phényle ; et R11 un radical —Ar$CO_2$R3, —Ar$SO_2$-(alcoyle en C1 à C6) ou —CH=CH—C($CH_3$)=CH$CO_2$R3 et R1, R3, Ar et X ont la signification donnée ci-dessus, et $Y^-$ est l'anion d'un acide inorganique ou organique,
et si on le désire en ce qu'on saponifie un ester d'acide carboxylique obtenu, en ce qu'on le transforme en un amide ou en ce qu'on le réduit en alcool et en ce que si on le désire on éthérifie celui-ci ou en ce qu'on l'oxyde en groupe formyle ou en ce qu'on transforme un acide carboxylique obtenu en un sel.

8. Procédé de préparation d'un rodenticide, caractérisé en ce qu'on fait réagir un composé de formule I de la revendication 1 ou un de ses sels avec les supports habituels.

9. Application de composés de formule I de la revendication 1 pour combattre les rongeurs nuisibles.